# EUROPÄISCHE PATENTANMELDUNG

(11) **EP 4 257 135 A1**
(43) Veröffentlichungstag der Anmeldung: **11.10.2023**
(21) Anmeldenummer: 22167330.4
(22) Anmeldetag: 08.04.2022
(51) Int. Cl.: A61K 31/5377, A61P 9/00, A61P 11/00

(54) **BENZIMIDAZOLDERIVATE BEI PULMONALER ARTERIELLER HYPERTONIE**

(71) Anmelder: Justus-Liebig-Universität Gießen, 35390 Gießen (DE)
(72) Erfinder: Savai Pullamsetti, Soni, 61231 Bad Nauheim (DE); Chelladurai, Prakash, 61231 Bad Nauheim (DE); Seeger, Werner, 35444 Biebertal (DE); Savai, Rajkumar, 61231 Bad Nauheim (DE)
(74) Vertreter: Stumpf, Peter

(57) **Zusammenfassung**

Die vorliegende Erfindung beschreibt einer Verbindung der Formel (II) sowie derer Metallkomplexe, Salze, Enantiomere, Enantiomerengemische, Diastereomere, Diastereomeren-gemische, Tautomere, Hydrate, Solvate, Racemate, Dimere oder Oligomere in einer medizinischen Formulierung gegen pulmonale arterielle Hypertonie (PAH) bei Menschen.

Dieser Wirkstoff ist das Benzimidazolderivat 2-[2-(3-Chloro-4-methoxyphenyl)ethyl]-5-(3,5-dimethyl-1,2-oxazol-4-yl)-1-[(S)-2-(morpholin-4-yl)propyl]-1H-benzimidazole)). Das Benzimidazolderivat 2-[2-(3-Chloro-4-methoxyphenyl)ethyl]-5-(3,5-dimethyl-1,2-oxazol-4-yl)-1-[(S)-2-(morpholin-4-yl)propyl]-1H-benzimidazole)) hat die Summenformel C₂₈H₃₃ClN₄O₃ und die CAS-Nummer 1613695-14-9. Synonyme sind SGC-CBP30, SGCCBP30 oder SGC CBP30.

## Beschreibung

Die vorliegende Erfindung betrifft eine von Benzimidazol abgeleitete Verbindung der allgemeinen Formel (II) und ihre Verwendung in der Medizin zur Behandlung von pulmonaler arterieller Hypertonie (Synonyme: PAH, pulmonale Hypertonie, PH, PHT, pulmonary artery hypertension, ICD-10: 127.0 und I27.2). Des Weiteren sind medizinische Formulierungen enthaltend die erfindungsgemäße Verbindung beschrieben. Die Erfinder haben durch die intensive Beschäftigung mit der Erkrankung PAH deren zugrundeliegenden Auslöser identifiziert und auf der Basis dieser wissenschaftlichen Erkenntnisse eine neue Therapieoption entwickelt. Das wesentliche Prinzip besteht darin, dass bestimmte Benzimidazolderivate an Histon-Acetyl-Transferasen (HAT, HATs) binden können. Bei einer Bindung der Benzimidazolderivate an HATs von Lungenzellen bewirken die Benzimidazolderivate einen positiven Effekt im Lungengewebe von Patienten mit PAH. Die Applikation einer medizinischen Formulierung, umfassend zumindest ein Benzimidazolderivat, ist also dazu geeignet, eine PAH zu therapieren.

Die Erkrankung PAH ist durch einen Anstieg des Blutdruckes im Lungenkreislauf und einem zunehmenden Gefäßwiderstand in den Lungenarterien gekennzeichnet. Es wird zwischen einer primären und einer sekundären PAH unterschieden. Beide Formen einer PAH zeichnen sich durch einen erhöhten Blutdruck in den Lungenarterien aus. Üblicherweise wird nur die primäre PAH als PAH bezeichnet, während die sekundäre PAH in der Fachliteratur meistens als PH abgekürzt wird. In dieser Schrift wird sowohl die primäre als auch die sekundäre pulmonale arterielle Hypertonie als PAH abgekürzt, da der erfindungsgemäße Wirkstoff für beide Formen geeignet ist.

Die primäre PAH ist eine idiopathische Vaskulopathie mit Hypertension, welche nur die Lungenblutgefäße betrifft. Bei der primären PAH ist die Ursache des Blutdruckanstieges und der Erhöhung des Gefäßwiderstandes unbekannt. Eine primäre PAH ist selten. Bei der weitaus häufigeren sekundären PAH werden der Blutdruckanstieg und die Erhöhung des Gefäßwiderstandes durch andere Krankheiten verursacht, beispielsweise durch chronisch obstruktive Lungenerkrankungen (COPD), Herzinsuffizienz, Lungenembolie, Lungenfibrose, Sarkoidose, Acquired Immune Deficiency Syndrome (AIDS), Acute Respiratory Distress Syndrome (ARDS, akutes Lungenversagen, akutes Atemnotsyndrom), Sichelzellanämie, Sklerodermie oder angeborene Herzfehler. Die sekundäre PAH (oder PH im eigentlichen Sinn) ist mit einem zugrundeliegenden kausalen Krankheitsgeschehen assoziiert.

Es existieren mehrere Unterformen einer PAH, beispielsweise die idiopathische pulmonalarterielle Hypertonie (IPAH), die hereditäre pulmonalarterielle Hypertonie (HPAH), auch familiäre pulmonale Hypertonie (FPAH) genannt, die arzneimittel- oder toxininduzierte PAH (DPAH), die assoziierte pulmonalarterielle Hypertonie (APAH), die pulmonale venookklusive Erkrankung (PVOD) oder pulmonalkapilläre Hämangiomatose (PCH) sowie die persistierende pulmonalarterielle Hypertonie des Neugeborenen (PPHN).

Eine sekundäre PAH kann durch Erkrankungen der linken Herzhälfte verursacht werden, beispielsweise bei einer systolischen oder diastolischen Dysfunktion, bei Mitral- oder Aortenklappenfehlern, bei angeborener oder erworbener Linksherz-Einfluss-/Ausflusstrakt-Obstruktion oder bei angeborenen Kardiomyopathien.

Eine sekundäre PAH kann auch durch Erkrankungen der Lunge verursacht werden, beispielsweise bei COPD, bei interstitieller Lungenerkrankung, bei anderen restriktiven und obstruktiven gemischten pulmonalen Erkrankungen, bei Schlafapnoe-Syndrom, bei alveolärer Hypoventilation, bei chronischer Höhenkrankheit oder bei anlagebedingten Fehlbildungen.

Eine sekundäre PAH kann auch durch Obstruktionen der Lungenarterien (Pulmonalarterien) verursacht werden, beispielsweise bei chronischthromboembolischer pulmonaler Hypertonie (CTEPH).

Eine sekundäre PAH kann auch durch multifaktorielle Mechanismen verursacht werden, beispielsweise bei einer chronisch-hämolytischen Anämie, bei myeloproliferativen Erkrankungen, bei Sarkoidose, bei pulmonaler Langerhans-Zell-Histiozytose, bei Neurofibromatose, bei einer Glykogenspeicherkrankheit, bei Morbus Gaucher, bei einer chronischen Niereninsuffizienz, bei einer fibrosierenden Mediastinitis sowie bei komplexen angeborenen Herzerkrankungen.

### Stand der Technik

Für die Therapie einer sekundären PAH ist es wichtig, ob die zugrundeliegende primäre Erkrankung therapierbar ist oder nicht. Bei einer sekundären PAH wird also vor allem die zugrundeliegende primäre Erkrankung behandelt. Oftmals verbessert sich dadurch auch die PAH. Weitere allgemeine therapeutische Maßnahmen sind eine Sauerstoff-Langzeittherapie (LTOT), eine ausreichende Versorgung mit Eisen, der Einsatz von Diuretika, der Einsatz von Antikoagulantien, angemessenes körperliches Training, der Verzicht auf das Rauchen und eine Gewichtsabnahme im Fall von Übergewicht.

Die spezifische medikamentöse Therapie der PAH ist schwierig. Folgende Arzneistoffe sind für die Therapie der PAH bekannt:
- Endothelin-Rezeptorantagonisten wie z. B. Ambrisentan, Atrasentan, Bosentan, Clazosentan, Macitentan, Sitaxentan oder Tezosentan;
- Phosphodiesterase-5-Hemmer (PDE-5-Hemmer) wie z.B. Coffein, Theophyllin, Theobromin, Sildenafil, Tadalafil, Vardenafil oder Avanafil;
- Riociguat;
- Prostacyclin-Analoga wie z.B. Epoprostenol, Iloprost oder Treprostinil;
- Calciumkanalblocker wie z.B. Dihydropyridine, Phenylalkylamine oder Benzothiazepine: Zu den Dihydropyridinen gehören Nitrendipin, Felodipin, Amlodipin, Nifedipin, Lercanidipin, Nimodipin, Nicardipin, Lacidipin, Isradipin, Nisoldipin, Nilvadipin, Manidipin und Clevidipin. Zu den Phenylalkylaminen gehören Verapamil und Gallopamil. Ein Vertreter der Benzothiazepine ist Diltiazem.

Allerdings weisen alle bekannten medikamentösen Therapieoptionen neben den verschiedenartigsten Nebenwirkungen eine nur mäßige Wirksamkeit gegen PAH auf, sodass in fortgeschrittenen Fällen als letzte Option nur eine Lungentransplantation verbleibt.

Die derzeitigen medikamentösen Therapien zur Behandlung der PAH zielen im Allgemeinen darauf ab, das Ungleichgewicht zwischen Vasodilatatoren und Vasokonstriktoren zu beseitigen und so die Symptome zu lindern und die Prognose dieser tödlichen Krankheit zu verbessern. Diese Therapien sind jedoch im Hinblick auf die Wiederherstellung der strukturellen und funktionellen Integrität der Lungengefäße, die die Grundlage für ein behinderungsfreies Langzeitüberleben bildet, begrenzt. Daher bleibt die Wiederherstellung der physiologischen Gefäßstruktur und -funktion eine Herausforderung. In den letzten Jahren wurden erhebliche Fortschritte beim Verständnis der molekularen Mechanismen erzielt, die dem pulmonalen Gefäßumbau bei PAH zugrunde liegen. Die meisten Untersuchungen im Stand der Technik konzentrierten sich auf die Identifizierung der wichtigsten auslösenden Signale und ihrer nachgeschalteten Effektoren, die den Ausbruch der PAH vorantreiben. Ist die Krankheit jedoch erst einmal voll ausgeprägt und sind die krankheitsrelevanten Zelltypen im Lungengefäßsystem dauerhaft aktiviert, dann ist die therapeutische Wirksamkeit von Eingriffen in diese Signalwege begrenzt. Vor diesem Hintergrund haben die Erfinder eine ex vivo-Phänotypisierung von Lungengefäßzellen durchgeführt, die aus menschlichen PAH-Lungen isoliert wurden. Anschließend untersuchten die Erfinder die zugrunde liegende transkriptionelle und epigenetische Landschaft eingehend, gefolgt von einem Integromik-Ansatz, der epigenomische und transkriptomische Analysen kombiniert, um Regulationsmechanismen und -wege zu entdecken und zu katalogisieren, die bei der Entwicklung von epigenombasierten therapeutischen Ansätzen helfen können.

Die inhalative Applikation ist eine Verabreichung von Wirkstoffen oder medizinischen Formulierungen in Form eines Aerosols über Inhalation in die Lunge. Sie wird auch pulmonale Applikation genannt. Sie ist eine geeignete Möglichkeit, um dem Patienten eine leicht anwendbare Verabreichung zu ermöglichen. Unerwünschte systemische Nebenwirkungen, die mit der intravenösen oder oralen Applikation verbunden sind, werden dem Patienten erspart. Weiterhin kann die eingesetzte Wirkstoffmenge deutlich reduziert werden, da bei der inhalativen Applikation der Wirkstoff direkt an die Lunge gebracht wird und nicht erst eine Verteilung und Verdünnung durch den ganzen Körper stattfindet. Besonders vorteilhaft ist die inhalative Applikation bei der Verabreichung von medizinischen Formulierungen zur Therapie von Lungenerkrankungen.

Das Europäische Arzneibuch unterscheidet folgende Zubereitungen, die zur inhalativen Applikation geeignet sind:
- Zubereitungen, die in Dampf überführt werden;
- Flüssige Zubereitungen zur Vernebelung;
- Flüssige Zubereitungen in Druckgas-Dosierinhalatoren
- Pulver zur Inhalation

Das Europäische Arzneibuch nennt als Geräte, die die Zubereitungen zur Inhalation verabreichen können:
- Vernebler
- Inhalatoren
- Druckgas-Dosierinhalator
- Normaldruck-Dosierinhalator
- Pulver-Inhalator

Die direkte inhalative Applikation des Wirkstoffs in die Lunge erleichtert die gezielte und nebenwirkungsarme Behandlung der pulmonalen Hypertonie. Als geeignete Wirkstoffträgersysteme für die inhalative Applikation sind biokompatible Nanopartikel wie beispielsweise Nanopartikel aus PLGA (Polylactid-co-Glycolid), PLA (Polylactide, Polymilchsäuren), PLA-PEO-PLA (Polylactide-Polyethylenoxid-Polylactide bzw. Poly(lactide)-block-Poly(ethylenoxid)-block-Poly(lactide)) bekannt. Alle bekannten medikamentösen Therapieoptionen gegen PAH weisen Mängel auf, vor allem wenn es darum geht, die physiologische Gefäßstruktur und -funktion einer bereits nachhaltig geschädigten Lunge wiederherzustellen. Daher besteht eine sehr große Notwendigkeit für die Entwicklung neuer Medikamente gegen PAH, die in erster Linie die physiologische Gefäßstruktur und -funktion einer bereits nachhaltig geschädigten Lunge wiederherstellen.

### Aufgabe

Die Aufgabe der vorliegenden Erfindung ist es, eine Verbindung als Wirkstoff zur Verfügung zu stellen, mit dem eine spezifischere und besser verträgliche Therapie der Erkrankung PAH bei Menschen und Säugetieren erreicht wird. Insbesondere soll eine Wiederherstellung der physiologischen Gefäßstruktur und -funktion in der Lunge erreicht werden. Dieser Wirkstoff wird mit geeigneten und dem Fachmann bekannten Zusatzstoffen kombiniert, um eine medizinische Formulierung zu erreichen, die einfach und sicher anwendbar ist, gut verträglich ist und eine gute Wirksamkeit aufweist.

Diese Aufgabe wird durch die technische Lehre der unabhängigen Ansprüche gelöst. Weitere vorteilhafte Ausgestaltungen, Aspekte und Details der Erfindung ergeben sich aus den abhängigen Ansprüchen, der Beschreibung und den Beispielen.

### Lösung der Aufgabe

Die Erfindung betrifft eine Verbindung der allgemeinen Formel (II) sowie deren Metallkomplexe, Salze, Enantiomere, Enantiomerengemische, Diastereomere, Diastereomerengemische, Tautomere, Hydrate, Solvate und Racemate der vorgenannten Verbindung. Die Verbindung der Formel (II) entsprechend der vorliegenden Erfindung kann selbst oder in Form eines pharmakologisch wirksamen Salzes verabreicht werden. Salze dieser Verbindung können nach gängigen Methoden hergestellt werden.

Insbesondere betrifft die Erfindung die Verwendung einer Verbindung der Formel (II) sowie derer Metallkomplexe, Salze, Enantiomere, Enantiomerengemische, Diastereomere, Diastereomerengemische, Tautomere, Hydrate, Solvate, Racemate, Dimere oder Oligomere in einer medizinischen Formulierung gegen pulmonale arterielle Hypertonie (PAH) bei Menschen, insbesondere einer idiopathischen pulmonalen arteriellen Hypertonie (IPAH).

Geeignete Beispiele dieser Salze der Verbindungen der Formel (II) umschließen Säureadditionssalze, Alkalimetallsalze sowie Salze mit Aminen. So können Alkalimetallsalze wie das Natriumsalz, das Kaliumsalz, das Lithiumsalz oder das Magnesiumsalz, das Calciumsalz, Alkylammoniumsalze oder Aminosäurensalze z.B. mit basischen Aminosäuren wie Lysin, genannt werden. Als Säuren, welche ein Säureadditionssalz der Verbindung der Formel (II) bilden, können die folgenden genannt werden: Schwefelsäure, Sulfonsäuren, Phosphorsäure, Salpetersäure, salpetrige Säure, Perchlorsäure, Bromwasserstoffsäure, Chlorwasserstoffsäure, Ameisensäure, Essigsäure, Propionsäure, Bernsteinsäure, Oxalsäure, Gluconsäure (Glyconsäure, Dextronsäure), Milchsäure, Apfelsäure, Weinsäure, Tartronsäure (Hydroxymalonsäure, Hydroxypropandisäure), Fumarsäure, Zitronensäure, Ascorbinsäure, Maleinsäure, Malonsäure, Hydroxymaleinsäure, Brenztraubensäure, Phenylessigsäure, (o-, m-, p-) Toluylsäure, Benzoesäure, p-Aminobenzoesäure, p-Hydroxybenzoesäure, Salicylsäure, p-Aminosalicylsäure, Methansulfonsäure, Ethansulfonsäure, Hydroxyethansulfonsäure, Ethylensulfonsäure, p-Toluolsulfonsäure, Naphthylsulfonsäure, Naphthylaminsulfonsäure, Sulfanilsäure, Camphersulfonsäure, Chinasäure (Chininsäure), o-Methyl-Mandelsäure, Hydrogenbenzolsulfonsäure, Pikrinsäure (2,4,6-Trinitrophenol), Adipinsäure, d-o-Tolylweinsäure, Aminosäuren wie Methionin, Tryptophan, Arginin und insbesondere saure Aminosäuren wie Glutaminsäure oder Asparaginsäure.

Ferner umfasst die vorliegende Erfindung eine Verbindung gemäß der allgemeinen Formel (I) zur Verwendung in der Medizin und besonders bevorzugt zur Behandlung einer PAH, insbesondere einer idiopathischen pulmonalen arteriellen Hypertonie (IPAH).

Ferner betrifft die vorliegende Erfindung medizinische Formulierungen, welche unter Verwendung mindestens einer erfindungsgemäßen Verbindung oder eines Salzes davon hergestellt werden.

Neben mindestens einer Verbindung der Formel (II) enthalten die medizinischen Formulierungen einen pharmakologisch verträglichen Träger, Hilfsstoff und/oder Lösungsmittel.

Die medizinischen Formulierungen können in Form von Tropfen, Mundspray, Nasenspray, Pillen, Tabletten, Filmtabletten, Schichttabletten, Zäpfchen, Gelen, Salben, Sirup, Inhalationspulvern, Granulaten, Suppositorien, Emulsionen, Dispersionen, Mikrokapseln, Kapseln, Puder oder Injektionslösungen hergestellt und verabreicht werden. Die erfindungsgemäßen medizinischen Formulierungen umfassen auch medizinische Formulierungen zur kontrollierten und/oder kontinuierlichen Freisetzung des Wirkstoffs wie beispielsweise Schichttabletten oder Mikroverkapselungen. Die medizinischen Formulierungen können also in fester oder flüssiger Form verwendet werden, oder in Pulverform.

Derartige galenische Formen sind unter anderem für die Inhalation oder die intravenöse, intraperitoneale, intramuskuläre, subkutane, mukokutane, orale, rektale, transdermale, topikale, bukkale, intradermale, intragastrale, intrakutane, intranasale, intrabukkale, perkutane oder sublinguale Verabreichung geeignet.

Als pharmakologisch verträgliche Träger können beispielsweise Lactose, Stärke, Sorbitol, Sucrose, Cellulose, Magnesiumstearat, Dicalciumphosphat, Calciumsulfat, Talk, Mannitol, Ethylalkohol und der gleichen eingesetzt werden. Puder als auch Tabletten können zu 5 bis 95% aus einem derartigen Träger bestehen. Ferner können den medizinischen Formulierungen noch Sprengmittel, Farbstoffe, Geschmacksstoffe und/oder Bindemittel zugesetzt werden.

Flüssige medizinische Formulierungen umfassen Lösungen, Suspensionen, Sprays und Emulsionen, beispielsweise Injektionslösungen auf Wasserbasis oder Wasser-Propylenglycol-Basis für parenterale Injektionen.

Für die Zubereitung von Suppositorien werden bevorzugt niedrigschmelzende Wachse, Fettsäureester und Glyceride eingesetzt.

Kapseln werden beispielsweise aus Methylcellulose, Polyvinylalkohole oder denaturierter Gelatine oder Stärke hergestellt.

Als Sprengmittel können Stärke, Natrium-Carboxymethylstärke, natürliche und synthetische Gummis wie beispielsweise Johannisbrotkernmehl, Karaya, Guar, Tragacanth und Agar sowie Cellulosederivate wie Methylcellulose, NatriumCarboxymethylcellulose, mikrokristalline Cellulose sowie Alginate, Tonerden und Bentonite verwendet werden. Diese Bestandteile können in Mengen von 2 bis 30 Gew.-% eingesetzt werden.

Als Bindemittel können Zucker, Stärke aus Mais, Reis oder Kartoffeln, natürliche Gummis wie Akaziengummi, Gelatine, Tragacanth, Alginsäure, Natriumalginat, Ammonium-Calcium-Alginat, Methylcellulose, Wachse, NatriumCarboxymethylcellulose, Hydroxypropyl-methylcellulose, Polyethylenglycol, Polyvinylpyrrolidon sowie anorganische Verbindungen wie Magnesium-Aluminum-Silicate zugesetzt werden. Die Bindemittel können in Mengen von 1 bis 30 Gew.-% zugesetzt werden.

Als Gleitmittel können Borsäure und Stearate wie Magnesiumstearat, Calciumstearat, Kaliumstearat, Stearinsäure, hochschmelzende Wachse als auch wasserlösliche Gleitmittel wie Natriumchlorid, Natriumbenzoat, Natriumacetat, Natriumoleat, Polyethylenglycol und Aminosäuren wie Leucin eingesetzt werden. Derartige Gleitmittel können in Mengen von 0,05 bis 15 Gew.-% verwendet werden. Die erfindungsgemäße Verbindung (Wirkstoff) und die oben beschriebenen medizinischen Formulierungen werden zur Behandlung von PAH eingesetzt. Dafür werden die dem Fachmann bekannten Grundlagen der Galenik eingehalten.

Die erfindungsgemäße Aufgabe wird gelöst durch eine medizinische Formulierung zur Verwendung bei Säugetieren, insbesondere bei Menschen, die an PAH erkrankt sind, umfassend mindestens ein Benzimidazolderivat mit der Grundstruktur 5-(3,5-Dimethyl-isoxazol-4-yl)-1H-benzimidazol. Die chemische Struktur ist als Formel (I) dargestellt.

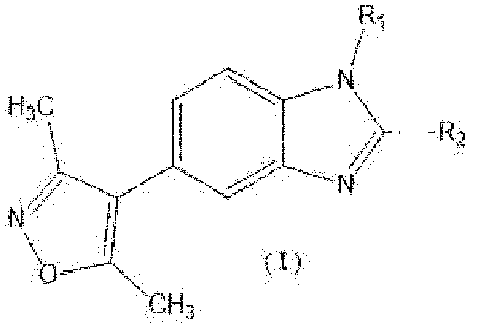

In einer bevorzugten Ausführungsform handelt es sich bei dem Benzimidazolderivat um 2-[2-(3-Chloro-4-methoxyphenyl)ethyl]-5-(3,5-dimethyl- -[(S)-2-(morpholin-4-yl)propyl]-1H-benzimidazole)). Diese hetero-zyklische Verbindung besteht aus einer Benzimidazolstruktur mit drei Resten. Das 2-[2-(3-Chloro-4-methoxyphenyl)ethyl]-5-(3,5-dimethyl-1,2-oxazol-4-yl)-1-[(S)-2-(morpholin-4-yl)propyl]-1H-benzimidazole)) hat die Summenformel C₂₈H₃₃ClN₄O₃ und die CAS-Nummer 1613695-14-9. Synonyme sind SGC-CBP30, SGCCBP30 oder SGC CBP30. Die chemische Struktur ist als Formel (II) dargestellt.

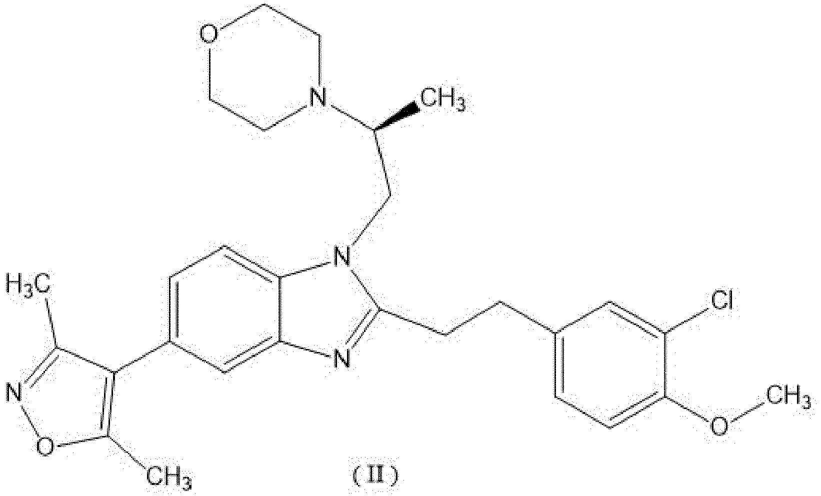

SGC-CBP30 ist ein bekannter Bromodomain-Inhibitor und hemmt spezifisch die beiden Histon-Acetyltransferasen EP300 und CREBBP. SGC-CBP30 ist ein kleines Molekül, das für den Laborbedarf kommerziell erhältlich ist. SGC-CBP30 zeigt eine epigenetische Wirkung bei Tumoren, neurodegenerativen Erkrankungen, Spondylitis ankylosans (Morbus Bechterew) und Arthritis psoriatrica (Psoriasisarthritis, PSA). SGC-CBP30 kann in einer geeigneten Zubereitungsform (Kapsel, Dragee, Tablette, Filmtablette, Pellet, Geleekapsel) einem Patienten oral verabreicht werden. Ein bevorzugter Hilfsstoff für die Herstellung einer oralen Verabreichungsform ist Methylcellulose. Eine geeignete Dosis für die orale Applikation ist 50 mg zweimal täglich (BD). Diese Dosis kann im Wechsel drei Tage lang verabreicht werden mit einer anschließenden Pause über 4 Tage. Das Behandlungsschema ist in diesem Fall 3 Tage Medikation mit 2 x 50 mg im Wechsel mit 4 Tagen ohne Medikation. Ein anderes geeignetes Behandlungsschema für die orale Therapie ist kontinuierlich 2 x 25 mg pro Tag (BD), also ohne behandlungsfreie Phasen. Ein anderes geeignetes Behandlungsschema für die orale Therapie ist kontinuierlich 2 x 10 mg pro Tag (BD), also ohne behandlungsfreie Phasen. Bei einer oralen Therapie wird ein Behandlungszeitraum von mindestens 14 Tagen empfohlen.

In einer bevorzugten Ausführungsform wird SGC-CBP30 durch intra-tracheale Vernebelung appliziert. Dafür wird SGC-CBP30 mit DMSO und PBS in einer Lösung eingesetzt. SGC-CBP30 kann in einer Konzentration von 275 nmol/kg in 4% DMSO in PBS verwendet werden. In einem geeigneten Behandlungsschema wird die intra-tracheale Vernebelung dreimal pro Woche über einen Zeitraum von zwei Wochen durchgeführt. Bei jeder einzelnen Behandlung mittels intra-trachealer Vernebelung werden 50 µl der SGC-CBP30-DMSO-PBS-Lösung eingesetzt.

Ebenfalls können andere, dem Fachmann bekannte Wirkstoffträgersysteme verwendet werden, um SGC-CBP30 inhalativ zu verabreichen.

### Ausführungsbeispiele

Zuerst führten die Erfinder next generation-DNA-Sequenzierungsverfahren bei pulmonalen Gefäßzellen durch, wobei hochgradig charakterisierte adventitiale Fibroblasten (FB, FBs) als Prototyp für eine PAH ex vivo verwendet wurden. Diese FB wurden von PAH-Patienten isoliert, um eine genomweite Perspektive auf gefäßzellspezifische Chromatinveränderungen, relevante Enhancerstellen, fehlregulierte Entwicklungs-Transkriptions-Faktoren (TF, TFs) und die damit verbundenen Transkriptionsziele zu erhalten, die der Pathobiologie des Gefäßumbaus bei PAH zugrunde liegen.

Überraschenderweise fanden die Erfinder, dass humane adventitiale Fibroblasten (FBs), die aus den Lungenarterien von PAH-Patienten isoliert wurden (PAH-FBs), durchwegs einen proproliferativen und antiapoptotischen Phänotyp aufweisen. Dieser krankheitsdefinierende Phänotyp blieb in vitro auch nach mehrfachen Passagen ohne komplexe in vivo-Umgebungseinflüsse erhalten, was auf zugrundeliegende genregulatorische Ereignisse und ein epigenetisches Gedächtnis hindeutet, die während der ex vivo-Passage nicht ohne weiteres reversibel sind. Darüber hinaus ergaben die auf Sequenzierung basierenden Ansätze 2.093 signifikante Differentially Expressed Genes (DEG, DEGs) bei der frühen Passage und 2.460 DEGs bei der späten Passage von PAH-FBs im Vergleich zu den entsprechenden FBs von gesunden Spendern (Spender-FBs). Da eine analoge Expression von 2.093 der 2.460 Gene zwischen frühen und späten Passagen beobachtet wurde, deutet dies nicht nur auf den minimalen Einfluss der Passage/Anpassung an die Kulturschalen auf die Genexpression hin, sondern bestätigt auch eine unverzichtbare Rolle der Genregulation bei der Aufrechterhaltung des PAH-Phänotyps.

Die Analyse der Chromatin-Immunpräzipitationsdatensätze zeigte eine umfangreiche unterschiedliche Verteilung von Histon-posttranslationalen Modifikationen (posttranslational modification, PTM, PTMs) in Verbindung mit transkriptionell zugänglichen Chromatinsignaturen (H3K9/K14ac, H4K5/8/12/16ac, H3K27ac und H3K4me3) auf der Promotorebene von 1.056 Genen (TSS ± 2 kb) in PAH-FBs im Vergleich zu denen in Spender-FBs. Wichtig ist, dass eine große Untergruppe der unterschiedlich transkribierten Gene signifikant mit einer spezifischen Anreicherung oder Verarmung von Histonacetylierungs- und Methylierungssignaturen und den daraus resultierenden Veränderungen der Transkriptionsaktivität korreliert.

Insbesondere die Identifizierung der umfangreichen genomweiten unterschiedlichen Verteilung des H3K27ac-Musters bei 2.858 Genen zwischen Spender-FBs und PAH-FBs war eine der wichtigsten Erkenntnisse dieser promotorspezifischen Analysen. Ausgehend von der Verteilungsprofile der Histon-Modifikationen H3K27ac-, H3K4me1- und H3K4me3 erkannten die Erfinder, dass 11.138 potenzielle transkriptionelle Enhancer-Regionen in PAH-FBs unterschiedlich modifiziert sind. Die Identifizierung von ausschließlich 2.327 spenderspezifischen und 1.825 PAH-spezifischen aktiven Enhancern (insgesamt 4.152 veränderte aktive Enhancer zwischen PAH-FB und Spender-FB) führte zu der Erkenntnis, dass eine genomweite Umgestaltung der Enhancer-Landschaft zu den pathologischen Transkriptionsprogrammen beiträgt, die den hypertensiven Phänotypen bei einer PAH zugrunde liegen. Neben der Ablagerung der Histon-Modifikation H3K27ac beobachteten die Erfinder umfangreiche genomweite Veränderungen der H3- und H4-Acetylierungsmuster (Histon-Modifikation H3K9/K14ac und H4K5/8/12/16ac) an einer Vielzahl von Genpromotoren, was auf eine abnorme Rekrutierung und erhöhte Aktivität von Histon-Acetyltransferasen (HATs) hindeutet. Tatsächlich ist H3K27 eines der wichtigsten Substrate, die von den beiden HATs EP300 und CREBBP in vivo acetyliert wird, und die Anreicherung von H3K27ac erleichtert die Öffnung des Chromatins und die Rekrutierung von Koaktivatoren, die ε-Acetyl-Lysin über eine Bromodomäne erkennen. Überraschenderweise wurde auch eine Hochregulierung der beiden HATs EP300 und CREBBP in PAH-FB beobachtet, was die unterschiedliche EP300-Acetyltransferase-Aktivität auf die genomweite Ablagerung von H3K27ac an Genpromotoren und Enhancerstellen bestätigt.

Ausgehend von diesen Erkenntnissen wurde in weiteren Untersuchungen gefunden, dass die Hemmung der HAT einen positiven Effekt auf eine PAH hat. Insbesondere wird durch die Hemmung der beiden HATs EP300 und CREBBP die physiologische Gefäßstruktur und -funktion einer bereits nachhaltig geschädigten Lunge wiederhergestellt. Die Substanz (Wirkstoff) SGC-CBP30 ist ein Bromodomain-Inhibitor und hemmt spezifisch die beiden HATs EP300 und CBP. Daher kann die Substanz SGC-CBP30 als Medikament bei einer PAH eingesetzt werden. Insgesamt zeigen die Untersuchungen der Erfinder, dass eine gezielte EP300-Hemmung und CREBBP-Hemmung durch die Substanz SGC-CBP30 die Auswirkungen der epigenetischen Dysregulation, die die Reaktivierung mesenchymaler Transkriptionsfaktoren und fetaler Transkriptionsprogramme bei erwachsener PAH vermittelt, weitgehend rückgängig machen können. In Anbetracht der Umkehrung der anhaltend aktivierten vaskulären Zellphänotypen und der Abschwächung der zugrundeliegenden aberranten Genexpressionsprogramme in Pulmonary Artery Smooth Muscle Cells (PASMCs) und Pulmonary Arterial Adventitial Fibroblasts (PAAFs), die ex vivo aus menschlichen und tierischen PAH-Modellen isoliert wurden, sowie des vaskulären Remodellings in menschlichen Precision-cut Lung Slices (PCLS) ex vivo, ist das therapeutische Potenzial der EP300-Hemmung und CREBBP-Hemmung vielversprechend, um das Remodelling der Lungengefäße bei schwerer PAH umzukehren.

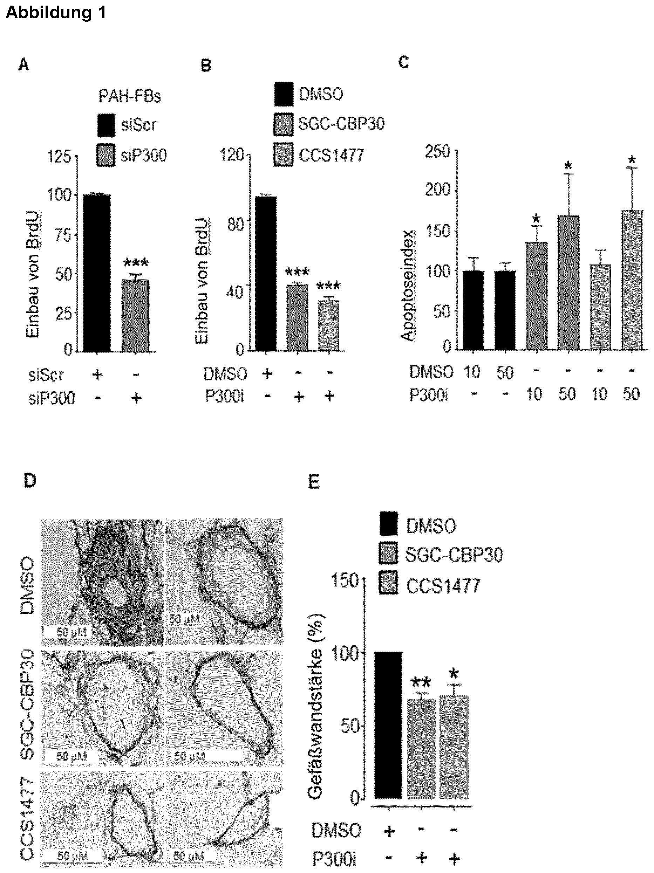

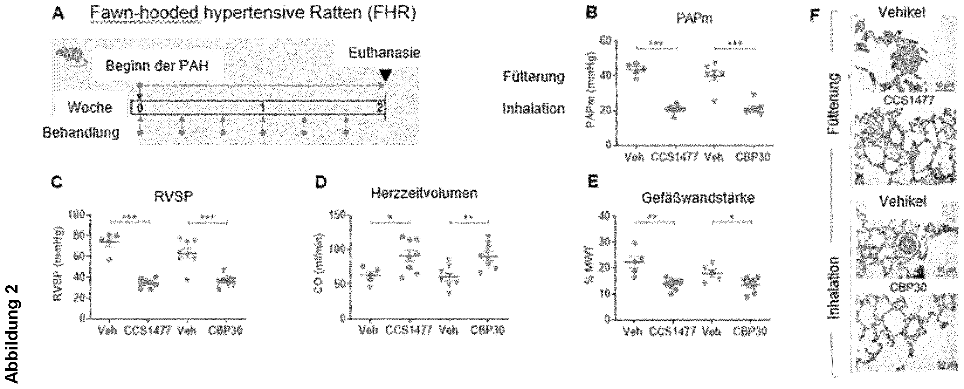

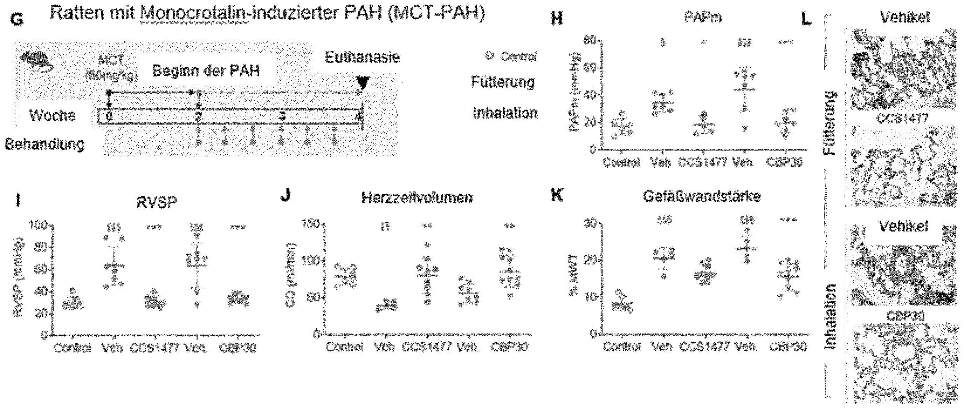

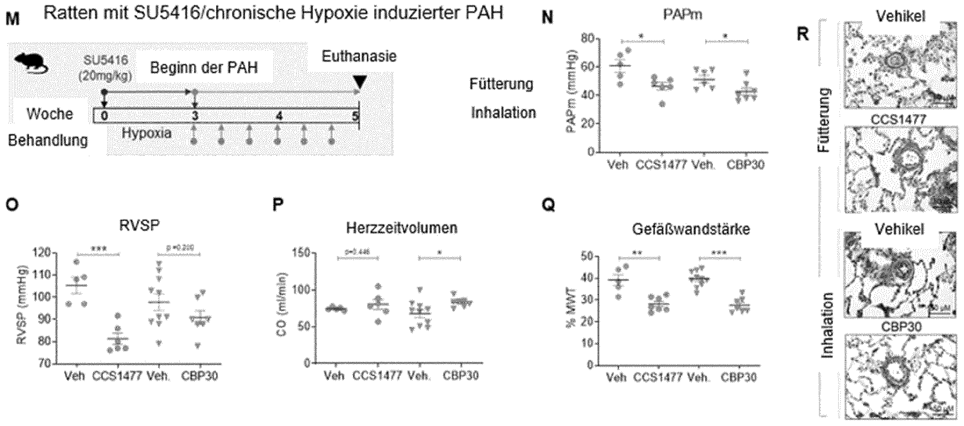

Das therapeutische Potenzial einer selektiven Beeinflussung epigenetischer Modifikatoren durch die Substanz SGC-CBP30 wurde sowohl ex vivo als auch in vivo untersucht (s. Abbildungen 1 und 2). Dabei wurden vor allem jene aberrant aktivierten molekularen Signaturen umgekehrt, welche den erworbenen phänotypischen Veränderungen in vaskulären Zellen zugrunde liegen, die mit schwerwiegenden vaskulären Umbauprozessen einhergehen und so die wichtigsten pathologischen Merkmale der PAH darstellen.

Ausgehend von der Erkenntnis, dass phänotypische Veränderungen in ortsansässigen Gefäßzellen zum Gefäßumbau bei kardiovaskulären Erkrankungen wie der PAH beitragen, welcher dann in einem rechtsventrikulären Herzversagen gipfelt, haben die Erfinder gezielt nach einem neuen Medikament gegen PAH gesucht. Wesentlich ist dabei das molekulare Zusammenspiel zwischen Transkriptions-Koaktivatoren, Transkriptionsfaktoren und Veränderungen des Chromatinzustands, welche durch abweichende Transkriptionsreaktionen die Aufrechterhaltung anhaltend aktivierter zellulärer Phänotypen ermöglicht. Dieser Prozess wurde durch die Erfinder gezielt erforscht: Die RNA-Sequenzierung (RNA-seq) in PAH-FBs aus erwachsenen menschlichen PAH-Patienten und in Spender-FBs aus gesunden Kontroll-Lungen ergab 2.460 differentiell transkribierte Gene. Die Chromatin-Immunpräzipitations-Sequenzierung (ChIP-seq) ergab eine umfangreiche unterschiedliche Verteilung von transkriptionell zugänglichen Chromatinsignaturen, wobei 4.152 aktive Enhancer in PAH-FBs verändert waren. Die integrative Analyse von RNA-seq- und ChIP-seq-Daten ergab eine starke Korrelation zwischen Signaturen posttranslationaler Modifikationen am Chromatin und dem Transkriptom. Die Transkriptionssignaturen für die Morphogenese der Lunge waren in PAH-FBs epigenetisch dereprimiert, einschließlich der Koexpression des T-Box Transkriptionsfaktors TBX4, des T-Box Transkriptionsfaktors TBX5 und des Transkriptionsfaktors SOX9, die an frühen Stadien der Lungenentwicklung beteiligt sind. Diese Transkriptionsfaktoren (TFs) wurden anfangs bei Mäuseföten stark exprimiert und postnatal unterdrückt, blieben aber bei persistierender PAH des Neugeborenen erhalten und wurden bei PAH von Erwachsenen wieder exprimiert. Die Ausschaltung von TBX4, TBX5, SOX9 oder EP300 durch RNA-Interferenz oder niedermolekulare Wirkstoffe führte zu einer Rückbildung des PAH-Phänotyps und der mesenchymalen Signaturen in arteriellen FBs und glatten Muskelzellen sowie zu einer Verringerung des Gefäßumbaus in PCLSs aus menschlichen PAH-Lungen ex vivo. Die pharmakologische Hemmung der HATs EP300 und CREBBP reduzierte signifikant den Umbau der distalen Lungengefäße, verbesserte die Hämodynamik und kehrte die etablierte PAH in drei Nagetiermodellen in vivo um. Die PAH-Pathogenese wird durch die epigenetische Reaktivierung von TFs beeinflusst, die mit der Lungenentwicklung verbunden sind. Daraus entwickelten die Erfinder den neuen therapeutischen Ansatz für die Verwendung von SGC-CBP30 als Wirkstoff gegen PAH.

Die Erfinder stellten die Vermutung auf, dass der persistente Phänotyp von PAH-Gefäßzellen, der für die Entwicklung komplexer Gefäßläsionen entscheidend ist, auf Veränderungen von Histonmodifikationsmustern und den damit verbundenen genomweiten epigenetischen Veränderungen beruht und damit durch die Substanz SGC-CBP30 beeinflussbar ist. Um diese Erfindung zu belegen, wurden folgende Untersuchungen durchgeführt:
1. Profilierung und Analyse der zelltypspezifischen transkriptomischen Unterschiede zwischen gesunden Personen und Patienten mit PAH;
2. Katalogisierung der genomweiten Veränderungen in der Histonacetylierung und Durchführung einer integrativen Analyse anhand von Genexpressionsprofilen;
3. Identifizierung und Erforschung der wichtigsten mit dem PAH-Phänotyp assoziierten TFs (Master-TFs);
4. Charakterisierung des Expressionsmusters von HATs in menschlichem Lungengefäßgewebe;
5. Bewertung des therapeutischen Potenzials der HAT-Modulation durch die Substanz SGC-CBP30 bei der Umkehrung des PAH-Phänotyps ex vivo und der etablierten PAH in vivo.

Diese Untersuchungen führten zu der Erkenntnis, dass die epigenetische Regulation der entwicklungsbedingten TF-Expression in der Lunge durch Modulation der EP300/CREBBP-Funktion rückgängig gemacht werden kann. Die Wirkung des niedermolekularen Inhibitors (Substanz, Wirkstoff) SGC-CBP30 auf die Proliferation von Gefäßzellen, die Apoptose sowie die Gen- und Proteinexpression wurde mit Hilfe geeigneter Assays nach der Transfektion untersucht. Nachdem Zellen zur Subkonfluenz gewachsen sind, wurde ihnen mit steigenden Konzentrationen der selektive EP300-Inhibitor SGC-CBP30 (Sigma, Katalognummer: SML1133) hinzugefügt. Die aus PAH-Patienten isolierten FBs wurden also mit dem isoformselektiven HAT-Inhibitor SGC-CBP30 behandelt. Gleichzeitig wurde den Zellen das Lösungsmittel DMSO (Sigma) mit entsprechenden Konzentrationen hinzugefügt.

Lungengewebeproben wurden von Patienten mit IPAH entnommen. Aus den mit 3% niedrigschmelzender Agarose gefüllten Lungenproben von Patienten mit IPAH wurden mit einem Vibratom (Leica Biosystems) Gewebeschnitte (400 µm dick) angefertigt. Die Lungengewebeschnitte wurden in DMEM-GlutaMAX-Medium mit 10 % fetalem Kälberserum, 1 % Penicillin/Streptomycin und 0,1 % Amphotericin B (Thermo Fisher Scientific) kultiviert, und die Behandlung wurde am zweiten Tag eingeleitet. Die Substanz SGC-CBP30 wurde in einer Konzentration von 200 µM an den Tagen 2 und 6 verabreicht. Am 10. Tag wurde das Gewebe in 4% Paraformaldehyd (Roti-Histofix; Carl Roth) fixiert und in Paraffin eingebettet. Die mediale Wanddicke wurde in allen Gefäßen mit einer Größe von 20-150 µm in den Lungenschnitten gemessen.

Die Hemmung von EP300/CREBBP in hyperproliferativen PAH-FBs (ex vivo isoliert) durch die Behandlung mit der Substanz SGC-CBP30 schwächte den hyperproliferativen Phänotyp und die Apoptoserate von PAH-FBs und PAH-SMCs deutlich ab. PAH-FBs wurden mit verschiedenen Konzentrationen der Substanz SGC-CBP30 oder dem Lösungsmittel DMSO inkubiert, um die Zellproliferation und die Induktion von Apoptose 24 Stunden danach zu untersuchen. Die Wirkung der selektiven EP300/CREBBP-Inhibition auf die Zellproliferation wurde durch den Einbau von BrdU (Bromodeoxyuridin, 5-Bromo-2'-Deoxyuridin, BUdR, BrdUrd, Broxuridine) bewertet. Die Wirkung der Substanz SGC-CBP30 oder des Lösungsmittels DMSO auf die Induktion von Apoptose wurde durch Cell Death Detection ELISAPLUS bewertet. Dabei zeigten sich deutliche Unterschiede zwischen der Substanz SGC-CBP30 und dem Lösungsmittel DMSO.

Das therapeutische Potenzial der EP300/CREBBP-Hemmung durch die Substanz SGC-CBP30 wurde untersucht. Dazu wurde die Wirksamkeit auf PAH-FBs und PAH-SMCs geprüft, die aus den Lungenarterien von menschlichen PAH-Patienten und aus den Lungenarterien von Ratten mit MCT-PAH (Rattenmodell mit Monocrotalin) isoliert wurden. Die therapeutische Bewertung der EP300/CREBBP-Hemmung ex vivo an PCLS, die aus den Lungen menschlicher PAH-Patienten gewonnen wurden, ergab eine signifikante Verringerung der medialen Wanddicke der umgestalteten PAs in den PAH-PCLS, die mit der Substanz SGC-CBP30 inkubiert wurden, im Vergleich zu den PAH-PCLS, die mit DMSO inkubiert wurden. Die Wirkung der EP300-Hemmung auf das vaskuläre Remodelling wurde durch Quantifizierung der prozentualen medialen Wanddicke in den mit der Substanz SGC-CBP30 inkubierten PCLS im Vergleich zu den mit DMSO inkubierten Kontrollen bewertet. Dabei zeigten sich deutliche Unterschiede zwischen der Substanz SGC-CBP30 und DMSO.

Um das therapeutische Potenzial der EP300/CREBBP-Hemmung in vivo weiter zu erforschen, haben die Erfinder Versuchstiere in drei verschiedenen Nagetiermodellen mit etablierter PAH mit der Substanz SGC-CBP30 behandelt, wobei SGC-CBP30 durch intra-tracheale Vernebelung über zwei Wochen lang dreimal wöchentlich verabreicht wurde. Es wurden folgende drei verschiedenen Nagetiermodelle für eine PAH (FHR-PAH, MCT-PAH und SuHx-PAH) getestet:
- Fawn-hooded hypertensive Ratten (FHR): Das ist ein genetischer Rattenstamm, bei dem PAH im Alter spontan auftritt und der ähnliche PAH-Anomalien aufweist, wie sie bei IPAH bei Menschen nach 40 Wochen auftreten.
- Durch Monocrotalin (MCT) induziertes PAH-Rattenmodell.
- Ein SUGEN5416/chronische Hypoxie (SuHx)-Rattenmodell für PAH, bei dem die Ratten eine einmalige Injektion von SUGEN5416 erhielten und anschließend einer chronischen Hypoxie (10% O₂) ausgesetzt wurden (SuHx-PAH).

Für die MCT-PAH wurden männlichen und weiblichen SD-Ratten (Sprague-Dawley-Ratten) subkutan 60 mg/kg MCT (Sigma, Katalog: C2401) injiziert, um PAH zu induzieren. Bei der SuHx-PAH erhielten SD-Ratten eine einmalige subkutane Injektion von SUGEN5416 (20 mg/kg) und wurden drei Wochen lang einer normobaren Hypoxie ausgesetzt, bevor sie für zwei Wochen an die Raumluft zurückkehrten. Zwei Wochen nach der MCT-Injektion bzw. drei Wochen nach der SUGEN5416-Injektion (sobald PAH festgestellt wurde) wurden die Ratten nach dem Zufallsprinzip in vier Gruppen eingeteilt und erhielten zwei Wochen lang dreimal wöchentlich entweder Vehikel (Verabreichung von 1 % Methylcellulose in destilliertem Wasser oder intratracheale Vernebelung von 4 % DMSO in PBS als Negativkontrolle), SGC-CBP30 (Verabreichung von 10 mg/kg in 1 % Methylcellulose + destilliertem Wasser) oder SGC-CBP30 (intratracheale Vernebelung von 275 nmol/kg in 4 % DMSO + PBS). Einjährige männliche und weibliche FHR-Ratten mit etablierter PAH wurden in dieselben vier Gruppen wie beim MCT-Modell eingeteilt und den selben Behandlungsmethoden unterzogen. Für die Vernebelung wurden die Ratten mit 3%igem Isofluran betäubt. Ein 16-Gauge-Katheter wurde in die Luftröhre eingeführt. Ein Vernebler (Aerogen) wurde an den Katheter angeschlossen, und für jede Behandlung wurde ein Volumen von 50 µl SGC-CBP30 oder dessen Vehikel (als Negativkontrolle) verabreicht. Nach zweiwöchiger Behandlung wurde eine Rechtsherzkatheteruntersuchung bei geschlossenem Thorax durchgeführt, um folgende hämodynamischen Daten zu erfassen: Systolischer Rechtsventrikulärer Druck (RVSP), mittlerer PA-Druck (mPAP), Herzzeitvolumen (CO) und Schlagvolumen (SV). Der Gesamtwiderstand der Lunge (TPR) wurde durch Division von mPAP durch CO ermittelt. Nach der hämodynamischen Messung wurden den Ratten Gewebeproben entnommen. Die Experimente wurden gemäß den Richtlinien des Canadian Council on Animal Care durchgeführt. Alle Protokolle wurden von der institutionellen Ethikkommission für Tiere (Tierschutzausschuss der Universität Laval) geprüft und genehmigt (Protokollnummer 2019-018-2). Alle Experimente folgten den Richtlinien für die präklinische Forschung bei PAH.

Die pharmakologische Hemmung von EP300/CREBBP mit der Substanz SGC-CBP30 führte zu einer signifikanten Senkung des RVSP, des mPAP und des TPR bei FHR-PAH, MCT-PAH und SuHx-PAH. Die kritischen Parameter zur Bewertung der RV-Funktion wie CO und SV verbesserten sich nach der Behandlung mit der Substanz SGC-CBP30 bei FHR-PAH und MCT-PAH signifikant. Die Substanz SGC-CBP30 verbesserte CO und SV auch bei SUGEN5416/chronischer Hypoxie-PAH signifikant. Die molekularen Marker für Myokardhypertrophie wurden durch die Substanz SGC-CBP30 in FHR-PAH signifikant abgeschwächt. Diese Verbesserung der relevanten Parameter einer PASH zeigt, dass die Substanz SGC-CBP30 das klinische Bild einer bestehenden PAH positiv beeinflusst.

In Übereinstimmung mit der signifikanten Reduktion der erhöhten Drücke RVSP, mPAP und TPR wurde die mediale Wanddicke der distalen PAs durch die pharmakologische EP300/CREBBP-Hemmung mit der Substanz SGC-CBP30 bei FHR-PAH, MCT-PAH und SuHx-PAH-Ratten signifikant verringert. Das Ergebnis dieser umfassenden Untersuchungen bestätigt die positiven therapeutischen Auswirkungen der pharmakologischen EP300/CREBBP-Hemmung durch die Substanz SGC-CBP30 auf die verbesserte Hämodynamik sowie die signifikante Umkehrung des pulmonalen Gefäßumbaus in vivo.

Um die Auswirkungen der EP300/CREBBP-Hemmung in vivo durch die Substanz SGC-CBP30 auf die Umkehrung der Genexpressionsmuster, die bei PAH beobachtet werden, zu bewerten, führten die Erfinder eine RNA-Sequenzierung des Lungengewebes von MCT-PAH-Ratten durch, die mit DMSO oder mit der Substanz SGC-CBP30 behandelt wurden, und fanden lungenweite transkriptionelle Veränderungen, die mit der EP300/CREBBP-Hemmung in Verbindung stehen. Die Analyse identifizierte 2279 DEGs in MCT-PAH-Ratten, die entweder mit der Substanz SGC-CBP30 oder mit DMSO behandelt wurden. Dazu gehörte die transkriptionelle Modulation der mesenchymal-epithelialen Übergangsfaktoren (MET-Faktoren) SOX9, PITX1 und PDE5A nach der Behandlung.

Die Substanz SGC-CBP30 verbessert die Hämodynamik signifikant und reduziert die Muskularisierung der distalen Lungengefäße durch Abschwächung der Zellzyklusprogression und der mesenchymalen Signaturen, die mit proproliferativen und apoptoseresistenten zellulären Phänotypen ex vivo und in den Nagetiermodellen der PAH in vivo verbunden sind.

### Abkürzungen und Definitionen

**ARDS:** akutes Lungenversagen, akutes Atemnotsyndrom, Acute Respiratory Distress Syndrome
**BD:** bis die, BID, bis in diem, Verabreichung eines Medikamentes zweimal täglich
**BrdU:** Bromodeoxyuridin, 5-Bromo-2'-Deoxyuridin, BUdR, BrdUrd, Broxuridine
**CCS1477:** (S)-1-(3,4-difluorophenyl)-6-(5-(3,5-dimethylisoxazol-4-yl)-1-((1r,4S)-4-methoxycyclohexyl)-1H-benzo[d]imidazol-2-yl)piperidin-2-on, Inobrodib, Inobrodibum, CCS-1477, CCS 1477, CBP-IN-1, CBP/p300-IN-4.
**ChIP-seq:** Chromatin-Immunpräzipitations-Sequenzierung
**CO:** Herzzeitvolumen, Cardiac Output; üblicherweise in Liter pro Minute angegeben
**COPD:** chronisch obstruktive Lungenerkrankung, Chronic Obstructive Pulmonary Disease
**CREBBP:** CBP, KAT3A, CREB-binding Protein, Cyclic Adenosine Monophosphate Response Element Binding Protein Binding Protein, AW558298, CBP/p300, p300/CBP, RSTS, RSTS1, MKHK1
**DEG, DEGs:** Differentially Expressed Gen, Differentially Expressed Genes
**DMSO:** Dimethylsulfoxid
**Enhancer** (Transkriptionsverstärker): DNA-Abschnitte, welche die Transkriptionsaktivität eines Gens verstärken. Enhancer-Landschaft ist die Gesamtheit der Enhancer im Genom.
**EP300:** Histon-Acetyltransferase P300, p300 HAT, E1A-assoziiertes Protein p300, KAT3B, RSTS2, E1A binding protein p300, MKHK2
**FB, FBs:** Fibroblast, Fibroblasten
**FHR:** Fawn-hooded hypertensive Ratten
**FHR-PAH:** PAH-Nagetiermodell mit Fawn-hooded hypertensive Ratten
**HAT, HATs:** Histon-Acetyltransferase, Histon-Acetyltransferasen
**H3K27:** Modifikation des Histons 3 durch Methylierung des Lysins 27
**IPAH:** Idiopathische Pulmonale Arterielle Hypertonie
**Kontrolle:** Control, mit DMSO behandelte Ratten als Kontrolle für die Wirkung des Wirkstoffes
**LTOT:** Sauerstoff-Langzeittherapie, Long Term Oxygen Therapy, Supplemental Oxygen, Oxygen Therapy
**MCT:** Monocrotalin (Alkaloid)
**MCT-PAH:** PAH-Nagetiermodell (Ratten), bei denen eine PAH mit Monocrotalin ausgelöst wird
**Medizinische Formulierung (Arzneiform, galenische Form):** Wirkstoff(e) und Hilfsstoff(e), die in einer geeigneten Form verarbeitet werden, um als Medikament verwendet zu werden
**MET:** Mesenchymaler-epithelialer Übergang, Mesenchymal-epithelial Transition
**mPAP:** PAPm, mittlerer Druck in der Pulmonalarterie, mittlerer PA-Druck, Mean Pulmonary Arterial Pressure
**MWT:** Gefäßwandstärke der Pulmonalarterien
**PA, PAs:** Pulmonalarterie, Pulmonalarterien
**PAAF, PAAFs:** Pulmonary Arterial Adventitial Fibroblast, Pulmonary Arterial Adventitial Fibroblasts, Fibroblasten der Pulmonalarterien
**PAH:** Pulmonale Arterielle Hypertonie, Pulmonale Hypertonie
**PAH-FB, PAH-FBs:** Fibroblast, Fibroblasten bei PAH-Patienten
**PAH-SMC, PAH-SMCs:** Smooth Muscle Cell, Smooth Muscle Cells, glatte Muskelzellen bei PAH-Patienten
**PAPm:** siehe mPAP
**PASMC, PASMCs:** Pulmonary Artery Smooth Muscle Cell, Pulmonary Artery Smooth Muscle Cells, glatte Pulmonalarterienmuskelzelle, glatte Pulmonalarterienmuskelzellen
**PBS:** Phosphate-buffered Saline, phosphatgepufferte Salzlösung
**PCLS:** Precision-cut Lung Slices, Lungengewebeproben, Präzisionslungenschnitte
**PDE5A:** cGMP-spezifische Phosphodiesterase Typ 5, Cyclic Guanosine Monophosphate-specific Phosphodiesterase Type 5, CGB-PDE, CN5A, PDE5, Phosphodiesterase 5A; wirkt als MET-Faktor
**PITX1:** BFT, CCF, LBNBG, POTX, PTX1, Paired Like Homeodomain 1; wirkt als MET-Faktor
**PTM, PTMs:** Posttranslational Modification, Posttranslational Modifications
**RNA-seq:** RNA-Sequenzierung, Sequenzierung der Ribonukleinsäure
**RV:** Rechter Herzventrikel, rechte Herzkammer, Right Ventricle
**RVSP:** Systolischer Rechtsventrikulärer Druck, Right Ventricular Systolic Pressure
**SD-Ratten:** Sprague-Dawley-Ratten
**SGC-CBP30:** 2-[2-(3-Chloro-4-methoxyphenyl)ethyl]-5-(3,5-dimethyl-1,2-oxazol-4-yl)-1-[(S)-2-(morpholin-4-yl)propyl]-1H-benzimidazole)), SGC-CBP30, SGCCBP30, SGC CBP30, CBP30
**SMC, SMCs:** Smooth Muscle Cell, Smooth Muscle Cells, glatte Muskelzellen
**SOX9:** Transkriptionsfaktor CMD1, CMPD1, SRA1, SRXX2, SRXY10, SRY-box 9, SRY-Box Transcription Factor 9; wirkt als MET-Faktor
**SuHx:** SUGEN5416 (VEGFR-Inhibitor)
**SuHx-PAH:** PAH-Nagetiermodell (Ratten), bei denen eine PAH mit SuHx in Kombination mit einer chronischen Hypoxie ausgelöst wird
**SV:** Schlagvolumen, Herzschlagvolumen, Stroke Volume
**TBX4:** T-Box Transkriptionsfaktor 4
**TBX5:** T-Box Transkriptionsfaktor 5
**TF, TFs:** Transkriptions-Faktor, Transkriptions-Faktoren
**TPR:** Gesamtwiderstand der Lunge, Total Peripheral Resistance
**Veh:** Vehikel, Vehicle; 1 % Methylcellulose in destilliertem Wasser bei Fütterung bzw. 4 % DMSO in PBS bei intratrachealer Vernebelung (Inhalation)
**Wirkstoff (Arzneistoff, Pharmakon):** Substanz, die in einem Organismus eine spezifische arzneiliche Wirkung hat

### Abbildungslegende

### Abbildung 1: Umkehrung von PAH-Phänotypen durch therapeutische Modulation der dysregulierten HATs EP300 und CREBBP ex vivo

**A:** PAH-FBs wurden mit siRNAs (siP300) behandelt, die auf EP300 abzielen (n=3), um die genspezifische funktionelle Rolle von EP300 bei der Hyperproliferation ex *vivo* 48 Stunden nach der Transfektion zu untersuchen. Die Zellproliferation wurde durch BrdU-Inkorporation bestimmt. Die Werte wurden auf scrambled siRNA (siScr) normalisiert und sind als Mittelwert ± SD angegeben (n=3, ungepaarter t-Test; *P < 0,05 vs. scrambled siRNA).
**B:** PAH-FBs wurden mit verschiedenen Konzentrationen des EP300/CREBBP-Inhibitors SGC-CBP30 oder dem Lösungsmittel DMSO inkubiert, um die Wirkung der selektiven EP300/CREBBP-Inhibition auf die Zellproliferation (bewertet durch BrdU-Inkorporation) zu untersuchen. Als Vergleichssubstanz wurde ein weiterer EP300/CREBBP-Inhibitor (CCS1477) parallel dazu unter denselben Bedingungen inkubiert. Die Werte wurden auf die DMSO-Kontrolle normiert und sind als Mittelwert ± SD angegeben (n=3, ungepaarter t-Test; *P < 0,05, **P < 0,01, ***P < 0,001 vs. DMSO).
**C:** PAH-FBs wurden mit verschiedenen Konzentrationen des EP300/CREBBP-Inhibitors SGC-CBP30 oder dem Lösungsmittel DMSO inkubiert, um die Wirkung der selektiven EP300/CREBBP-Inhibition auf die Induktion von Apoptose (bewertet durch Cell Death Detection ELISAPLUS) 24 Stunden nach der Inkubation zu untersuchen. Als Vergleichssubstanz wurde ein weiterer EP300/CREBBP-lnhibitor (CCS1477) parallel dazu unter denselben Bedingungen inkubiert. Die Werte wurden auf die DMSO-Kontrolle normiert und sind als Mittelwert ± SD angegeben (n=3, ungepaarter t-Test; *P < 0,05, **P < 0,01, ***P < 0,001 vs. DMSO).
**D:** Um den Beitrag der selektiven EP300/CREBBP-Hemmung zur Umkehrung der umgebildeten Lungengefäße in Lungen aus menschlicher PAH weiter zu bewerten, wurden aus sezierten PAH-Lungengeweben (n=3) *ex vivo* präzisionsgeschnittene Lungenscheiben (PCLS) erzeugt und mit dem EP300/CREBBP-Inhibitor SGC-CBP30 inkubiert. Als Kontrolle dient die Inkubation mit dem Lösungsmittel DMSO. Als Vergleichssubstanz wurde ein weiterer EP300/CREBBP-Inhibitor (CCS1477) parallel dazu unter denselben Bedingungen inkubiert. Hier werden repräsentative Bilder gezeigt. Skalenbalken: 50 µm.
**E:** Die Wirkung der P300-Inhibition (200 µM) auf den Gefäßumbau wurde durch Quantifizierung der prozentualen medialen Wanddicke in den mit dem EP300/CREBBP-Inhibitor SGC-CBP30 inkubierten PCLS im Vergleich zu den mit DMSO inkubierten Kontrollen bewertet. Als Vergleichssubstanz wurde ein weiterer EP300/CREBBP-Inhibitor (CCS1477) parallel dazu unter denselben Bedingungen inkubiert. Auch hier wurde der Gefäßumbau durch Quantifizierung der prozentualen medialen Wanddicke bewertet. Die Werte wurden auf die DMSO-Kontrolle normiert und sind als Mittelwert ± SD dargestellt (n=3, ungepaarter t-Test; *P < 0,05, **P < 0,01, ***P < 0,001 vs. DMSO).

### Abbildung 2: Umkehrung der Erkrankung PAH in verschiedenen Nagetiermodellen in vivo

Der schwarze Balken zeigt 50 µm an. Die Daten sind als Mittelwert ± SD angegeben und die statistische Analyse wurde mit einem ungepaarten Student's t-Test durchgeführt. FHR, MCT und SU5416/chronische Hypoxie-Ratten: *P<0,05, **P<0,01 und ***P<0,001 gegenüber den mit Vehikel behandelten Gruppen. MCT-Rattenmodell: §P<0,05, §§P<0,01 und §§§P<0,001 gegenüber der unbehandelten Kontrollgruppe.
**A:** Schematische Darstellung des Versuchsaufbaus für die Behandlung von Fawn-hooded hypertensive Ratten (FHR) mit dem EP300/CREBBP-Inhibitor SGC-CBP30, der selektiv auf die Acetyltransferase EP300/CREBBP wirkt. Als Vergleichssubstanz wurde ein weiterer EP300/CREBBP-Inhibitor (CCS1477) parallel dazu unter denselben Bedingungen eingesetzt.
**B:** Nach der Behandlung wurden die Fawn-hooded hypertensive Ratten einer Rechtsherzkatheteruntersuchung unterzogen, um den Parameter mittlerer Lungenarteriendruck (PAPm) zu analysieren.
**C:** Nach der Behandlung wurden die Fawn-hooded hypertensive Ratten einer Rechtsherzkatheteruntersuchung unterzogen, um den Parameter rechtsventrikulärer systolischer Druck (RVSP) zu analysieren.
**D:** Nach der Behandlung wurden die Fawn-hooded hypertensive Ratten einer Rechtsherzkatheteruntersuchung unterzogen, um den Parameter Herzzeitvolumen (CO) zu analysieren.
**E:** Nach der Behandlung wurden die Fawn-hooded hypertensive Ratten einer Rechtsherzkatheteruntersuchung unterzogen, um den Parameter prozentuale mediale Wanddicke zu analysieren.
**F:** Nach der Behandlung wurden von den Fawn-hooded hypertensive Ratten Lungengewebeschnitte angefertigt, um den Parameter Elastica-van Gieson (EvG)-Färbung zu analysieren.
**G:** Schematische Darstellung des Versuchsaufbaus für die Behandlung von Ratten, welche eine durch Monocrotalin (MCT) induzierte PAH aufweisen (MCT-PAH), mit dem EP300/CREBBP-Inhibitor SGC-CBP30, der selektiv auf die Acetyltransferase EP300/CREBBP wirkt. Als Vergleichssubstanz wurde ein weiterer EP300/CREBBP-Inhibitor (CCS1477) parallel dazu unter denselben Bedingungen eingesetzt.
**H:** Nach der Behandlung wurden die Ratten mit MCT-PAH einer Rechtsherzkatheteruntersuchung unterzogen, um den Parameter mittlerer Lungenarteriendruck (PAPm) zu analysieren.
**I:** Nach der Behandlung wurden die Ratten mit MCT-PAH einer Rechtsherzkatheteruntersuchung unterzogen, um den Parameter rechtsventrikulärer systolischer Druck (RVSP) zu analysieren.
**J:** Nach der Behandlung wurden die Ratten mit MCT-PAH einer Rechtsherzkatheteruntersuchung unterzogen, um den Parameter Herzzeitvolumen (CO) zu analysieren.
**K:** Nach der Behandlung wurden die Ratten mit MCT-PAH einer Rechtsherzkatheteruntersuchung unterzogen, um den Parameter prozentuale mediale Wanddicke zu analysieren.
**L:** Nach der Behandlung wurden von den Ratten mit MCT-PAH Lungengewebeschnitte angefertigt, um den Parameter Elastica-van Gieson (EvG)-Färbung zu analysieren.
**M:** Schematische Darstellung des Versuchsaufbaus für die Behandlung von Ratten mit SU5416/chronische Hypoxie induzierter PAH mit dem EP300/CREBBP-Inhibitor SGC-CBP30, der selektiv auf die Acetyltransferase EP300/CREBBP wirkt. Als Vergleichssubstanz wurde ein weiterer EP300/CREBBP-Inhibitor (CCS1477) parallel dazu unter denselben Bedingungen eingesetzt.
**N:** Nach der Behandlung wurden die Ratten einer Rechtsherzkatheteruntersuchung unterzogen, um den Parameter mittlerer Lungenarteriendruck (PAPm) zu analysieren.
**O:** Nach der Behandlung wurden die Ratten einer Rechtsherzkatheteruntersuchung unterzogen, um den Parameter rechtsventrikulärer systolischer Druck (RVSP) zu analysieren.
**P:** Nach der Behandlung wurden die Ratten einer Rechtsherzkatheteruntersuchung unterzogen, um den Parameter Herzzeitvolumen (CO) zu analysieren.
**Q:** Nach der Behandlung wurden die Ratten einer Rechtsherzkatheteruntersuchung unterzogen, um den Parameter prozentuale mediale Wanddicke zu analysieren.
**R:** Nach der Behandlung wurden von den Ratten mit SU5416/chronische Hypoxie induzierter PAH Lungengewebeschnitte angefertigt, um den Parameter Elastica-van Gieson (EvG)-Färbung zu analysieren.

## Patentansprüche

1. Verwendung einer Verbindung der Formel (II) sowie derer Metallkomplexe, Salze, Enantiomere, Enantiomerengemische, Diastereomere, Diastereomeren-gemische, Tautomere, Hydrate, Solvate, Racemate, Dimere oder Oligomere in einer medizinischen Formulierung gegen pulmonale arterielle Hypertonie (PAH) bei Menschen.

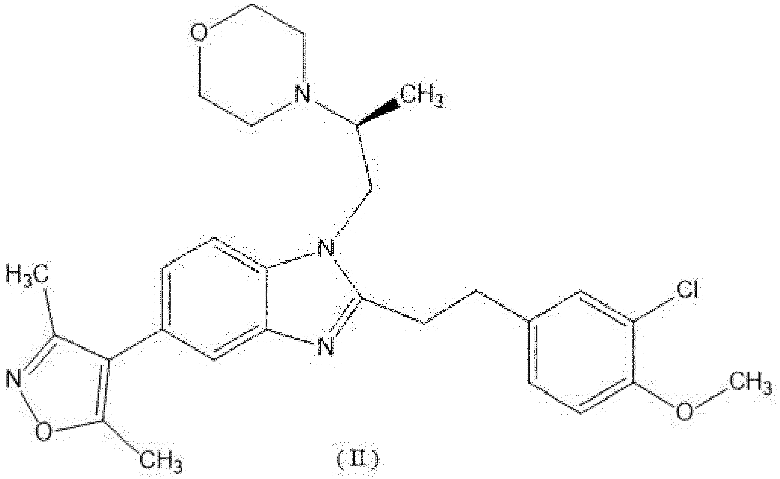

2. Medizinische Formulierung enthaltend mindestens die Verbindung der allgemeinen Formel (II) gemäß des Anspruchs 1 und mindestens einen pharmakologisch verträglichen Träger, Hilfsstoff und/oder Lösungsmittel.

3. Medizinische Formulierung gemäß Anspruch 2 in Form von Tropfen, Mundspray, Nasenspray, Pillen, Tabletten, Filmtabletten, Schichttabletten, Zäpfchen, Gelen, Salben, Sirup, Inhalationspulvern, Inhalationslösungen, Granulaten, Suppositorien, Emulsionen, Dispersionen, Mikrokapseln, Kapseln, Puder oder Injektionslösungen.

4. Medizinische Formulierung gemäß Anspruch 2 oder 3, **dadurch gekennzeichnet, dass** sie durch Inhalation oder intravenös, intraperitoneal, intramuskulär, subkutan, mukokutan, oral, rektal, transdermal, topikal, bukkal, intradermal, intragastral, intrakutan, intranasal, intrabukkal, perkutan oder sublingual verabreicht wird.

5. Medizinische Formulierung gemäß einer der vorangehenden Ansprüche, **dadurch gekennzeichnet, dass** die Verbindung nach Formel (II) für die orale Applikation in einer Konzentration von 10 mg/kg bzw.1 bis 10 mg/kg pro Tag eingesetzt wird.

6. Medizinische Formulierung gemäß Ansprüche 1 bis 4, **dadurch gekennzeichnet, dass** die Verbindung nach Formel (II) für die inhalative Applikation in einer Konzentration von 275 nmol/kg pro Tag verabreicht wird.

7. Medizinische Formulierung gemäß Anspruch 5 oder 6, **dadurch gekennzeichnet, dass** die Verbindung nach Formel (II) über einen Zeitraum von 14 Tagen verabreicht wird.
